# EUROPEAN PATENT SPECIFICATION

(11) **EP 3 209 337 B1**
(45) Date of publication and mention of the grant of the patent: **31.03.2021**
(21) Application number: 14815949.4
(22) Date of filing: 21.10.2014
(51) Int. Cl.: A61K 51/12, A61P 35/00, A61P 9/10, A61K 103/32, C01B 25/32, C03C 3/14, C03C 12/00, C03C 23/00, C04B 35/515, C04B 35/653, B01J 13/02

(54) **STRONTIUM PHOSPHATE MICROPARTICLE FOR RADIOLOGICAL IMAGING AND THERAPY**
STRONTIUM-PHOSPHAT-MIKROPARTIKEL FÜR RADIOLOGISCHE BILDGEBUNG UND THERAPIE
MICROPARTICULES DE PHOSPHATE DE STRONTIUM POUR L'IMAGERIE ET LA THÉRAPIE RADIOLOGIQUES

(43) Date of publication of application: 30.08.2017
(73) Proprietor: MO-SCI Corporation, Rolla, Missouri 65401 (US)
(72) Inventor: DAY, Delbert E., Rolla, Missouri 65401 (US); HE, Yiyong, Rolla, Missouri 65401 (US)
(74) Representative: Peterreins Schley
(86) International application number: PCT/US2014/061597
(87) International publication number: WO 2016/064379

(56) References cited:
- WO-A1-00/29034
- WO-A1-2005/087274
- US-A- 5 011 797
- US-B1- 6 537 518
- SINOUH H ET AL: "Effect of TiOand SrO additions on some physical properties of 33NaO-SrO-TiO-(50 - 2)BO", JOURNAL OF THERMAL ANALYSIS AND CALORIMETRY, KLUWER ACADEMIC PUBLISHERS, DORDRECHT, NL, vol. 111, no. 1, 28 March 2012 (2012-03-28), pages 401-408, XP035161385, ISSN: 1572-8943, DOI: 10.1007/S10973-012-2394-3

## Description

The field of the present invention is radiomicroparticles for medical therapy and imaging, and particularly radioactive microparticles comprising strontium phosphate, for radiological imaging and radioisotope therapy.

In the treatment of patients with certain kinds of cancer or rheumatoid arthritis, methods are known in which radioactive particles are introduced intravascularly to a tumor site (radioembolization) or locally into the synovial fluid in a joint in order to trap the radioactive particle at a particular site for its radiation effect. Similar methods are used for imaging parts of the body, organs, tumors, and so forth.

According to this technique, a quantity of the radioactive particles are injected into a localized area of the patient to be imaged and/or treated. For imaging, gamma emitting materials are commonly used to label carriers that provide imaging of a tissue area, tumor or organ. Some of these carriers have a specific affinity for certain binding sites or biochemical targets allowing target specific or location specific uptake of the labelled carrier.

Radiological imaging of various tissues in the human body is commonly accomplished using Technetium-99m, typically by single photon emission computed tomography (SPECT). ⁹⁹m_ Tc is a well-known radioactive isotope used for radio diagnostics such as SPECT. It emits detectable low level 140 keV gamma rays, has a half life of 6 hours and decays to Tc-99 in 24 hours (93.7%). It is used for imaging and function studies of the brain, myocardium, thyroid, lungs, liver, gallbladder, kidneys, bone, blood, and tumors. It is reported to be used in over 20 million diagnostic nuclear medicine procedures each year. Positron emission tomography (PET) employs radionuclides that emit positrons, a beta-like nuclear particle that travels a few millimetres from its nucleus, collides with an electron leading to annihilation resulting in creating two photons of 511 KeV that travel in 180° opposite direction. The PET imaging system captures and registers the photons arising from the collision precisely at the same time thereby providing exceptional imaging sensitivity. PET imaging has become a valuable diagnostic imaging procedure, particularly in the oncology area and it has been reported that in the US approximately two million PET scans are performed annually. Radioisotopes commonly employed for PET imaging include fluorine-18 (¹⁸F) that has a half-life of 109.8 minutes and gallium-68 (⁶⁸Ga) that has a half-life of 68 minutes.
Targeted radiation therapy using microparticles or microspheres is also a well developed field radioisotope therapy. Radionuclides such as Yttrium-90 and Holmium-166 are commonly used radioactive beta emitters in microsphere radiotherapy. Polymer microspheres such as albumin, poly-lactic acid derivatives, and so forth, and glass microspheres, are both generally known in the medical arts for use in delivering both pharmaceuticals and radiopharmaceuticals to specific tissue sites. These microspheres are usually provided preloaded with a single radionuclide and so lack the flexibility to control dose or radionuclide depending on the patient's needs. Further, when radio microparticles are prepared in bulk, off-site by third party providers, the selection of radionuclide available for use may be limited, by the time involved in preparation and delivery.

WO 00/29034 A1 discloses radioactive sources suitable for use in brachytherapy comprising one or more insoluble salts, wherein the insoluble salt contains, or the insoluble salts together contain, at least two different radioisotopes.

Thus a need remains for an improved radioactive microparticle for delivery of one or more radiopharmaceuticals and which have characteristics which will permit the microparticles to be suitable for injection into a patient for localized imaging or therapy.

In accordance with the present invention, novel, porous microparticle carriers have been devised for use in the imaging and/or treatment of certain cancers, tumor bearing tissue, rheumatoid arthritis, or other diseases where nuclear medicine imaging or treatment is indicated. These carriers constitute microparticles that comprise a porous strontium-phosphate material having one or more radiopharmaceuticals bound to the surface. Radio diagnostic gamma and positron emitting agents and radio therapeutic alpha and beta emitting agents are contemplated.

More specifically, the invention provides a radio microparticle comprising crystalline strontium phosphate at least the surface layer of which consists of non-radioactive crystalline strontium phosphate and at least one radioisotope suitable for radio imaging and/or radiotherapy bonded or adsorbed to the surface thereof.

The radio microparticle of the present invention is prepared by reacting a strontium-containing borate glass microparticle with a phosphate solution in amounts and for a sufficient time under suitable conditions (such as time, temperature, phosphate concentration etc.) to convert at least a portion strontium-containing borate glass at the surface to crystalline strontium phosphate, and bonding at least one radioisotope suitable for radio imaging and/or radiotherapy to the surface of said microparticle. This results in radiopaque, porous particles capable of being loaded with one or more radioisotopes and therefore being able to deliver radiation in a dose suitable for radiotherapy, or depending on the isotope chosen, for use in radiodiagnostics.

The invention also provides processes for preparing such radiomicroparticles. The invention also provides such radiomicroparticles for use in methods of medical treatment.

The invention therefore also provides a process for the preparation of a strontium, phosphate radio microparticle, comprising:
reacting a strontium-containing borate glass microparticle with a phosphate solution such as to convert at least a portion of the strontium-containing borate glass to crystalline strontium phosphate; and
bonding or adsorbing at least one radioisotope suitable for radio imaging and/or radiotherapy to said microparticle.

Conveniently the radioisotope is reacted with the strontium phosphate microparticle in the form of a solution, particulalry and aqueous solution. The radio isotope is conveniently in the form of a soluble salt. It is preferred that the salt should be soluble at least to the degree to allow the radioisotope to become bound to the surface of the microparticle and the reaction to proceed at a reasonable rate to prepare a therapeutically or diagnostically useful microparticle.

In other preferred embodiments, there are provided additional features available singularly and in combination.

Some of the advantages provided by this approach include: the ability to adsorb a radioisotope or combination of different radioisotopes onto a porous microparticle, allowing adaptability of the treatment, the ability to customize dose to the patient, customize imaging of the tissue, reducing time-related degradation of the activated radiopharmaceutical, and reducing exposure to medical personnel. Further advantages include the ability to use radioisotopes that have a short half-life and to avoid using microparticle manufacturing processes that would vaporize certain radioisotopes such as Technetium and Rhenium. These microparticles also display an improved radiopacity over previous calcium apatite particles which provides clearer radiographic images.

The phosphate solution conversion process converts solid strontium borate glass into a porous strontium phosphate material of the Bevolite Sr₁₀(PO₄)₆(OH)₂ type.

By manufacturing a non-radioactive solid strontium-containing borate glass microparticle of a specific diameter, the conversion results in a porous strontium phosphate microparticle of a specific diameter. Due to the substantially thorough chemical action of the phosphate solution on the borate glass, a substantially pure porous strontium phosphate material having a high surface area is achieved where the phosphate solution has reacted with the borate glass.

The strontium-containing borate glass microparticle is preferably a microsphere and preferably has a diameter of about 5 to about 1000µm. The strontium phosphate microparticle is also preferably a microsphere. The strontium-containing borate glass microparticle may be fully converted to a strontium phosphate microparticle, or it may be partially converted to create a layer comprising strontium phosphate covering the surface of the particle and an unconverted strontium containing borate glass core. The strontium phosphate containing portion is preferably porous and is crystalline. Preferably the strontium phosphate containing surface layer is at least about 0.5 µm thick, but it may be at least 1, 2, 3, 4, 5, 7 or 10 µm thick depending on the properties, such as binding capacity, density *etc.* required. The degree of conversion will depend on the reaction conditions, such as temperature, pH, concentration of the phosphate solution, time of reaction and so on and optimum conditions can readily be determined by those skilled in the art. In some embodiments, the strontium-containing borate glass microparticle may be substantially calcium-free.

The porosity of the resulting strontium phosphate material and the controllable size and number of the microparticles provide an excellent delivery platform for delivering compounds of interest to specific locations. Thus, the process of manufacturing of the microsphere has been divorced from the process of adding the radiolabel or radio therapeutic. This provides nuclear medicine professionals the ability to control the radio diagnostic and radio therapeutic regimen by allowing, in the clinical setting, at or near the time of delivery, the decision of the type and quantity of radiopharmaceutical(s) to be incorporated into the delivery vehicle.

Due to the porous nature of the strontium phosphate component, the microparticle has a greatly increased surface area. Preferably the surface area of the strontium phosphate microparticle is greater than about 90 square meters per gram and may be up to about 200 square meters per gram or greater.

The strontium phosphate microparticle comprises at least one radioisotope, suitable for radio imaging and/or radiotherapy. The microparticle may advantageously comprise one, two, three or more different radioisotopes. In one preferred embodiment, the radioisotope is, or comprises a therapeutic alpha or beta-emitting radioisotope or a diagnostic gamma or positron -emitting radioisotope. The radioisotope may also be, or comprise a combination of therapeutic alpha or beta-emitting radioisotope or diagnostic gamma or positron-emitting radioisotopes.

In a preferred embodiment, the radioisotopes / radionuclides are chosen so that, when administered to the patient, the microparticles emit either beta radiation, gamma radiation, or both. The beta emitter is chosen to deliver a therapeutic intensity and therapeutic amount of short-range (e.g., a penetration of the tissue in the order of about several millimetres or less) beta radiation but does not emit a significant amount of unwanted beta radiation which could have a negative impact on healthy tissue surrounding the cancerous or tumor bearing tissue. The gamma emitter is chosen to deliver a diagnostic intensity and diagnostic amount of longer-range (e.g., capable of external detection) gamma radiation but does not emit a significant amount of unwanted gamma radiation.

Since the radioisotopes / radionuclides are bonded or prepared in situ just prior to delivery by a radiology professional, the type of radioisotope(s) may be chosen based on each patient's needs and diagnosis, in a manner less limited by considerations of half-life. By providing a patient-specific dosing, patient outcome is improved and side-effects are minimized.

Patient data such as age, gender, weight, and pre-existing conditions are considered when determining a radio therapeutic and/or radio diagnostic profile. Cancer data such as tumor size, tumor type, tumor location, degree of surgical intervention and success, vascular structures within and adjacent to the area being treated, and organ involvement are also considered when determining a radio therapeutic and/ or radio diagnostic profile.

The radioisotope Yttrium-90, which forms radioisotopes having a half-life greater than about two days and less than about 30 days, is one particularly preferred therapeutic radioisotope which emit therapeutic beta radiation. For radio imaging techniques such as SPECT the radioisotope Technetium-99m is particularly preferred and for PET imaging Fluorine-18 or Gallium-68 are preferred.

The radioisotope is preferably of radiopharmaceutical grade and is selected from the group consisting of: Actinium-225, Antimony-127, Arsenic-74, Barium-140, Bismuth-210, Califomium-246, Calcium-46, Calciurn-47, Carbon-11, Carbon-14, Cesiurn-131, Cesium-137, Chromium-51, Cobalt-57, Cobalt-58, Cobalt-60, Copper-64, Copper-67, Dysprosium-165, Erbiurn-169, Fluorine-18, Gallium-67, Gallium-68, Gold-198, Holmium-166, Hydrogen-3, Indium-111, Indium-113m, Iodine-123, Iodine-124, Iodine-125, Iodine-131 (which may be used either as a diagnostic or a therapeutic isotope), Iridium-192, Iron-59, Iron-82, Krypton 81m, Lanthanum-140, Lutetium-177, Molybdemmi-99, Nitrogen-13, Oxygen-15, Paladium-103, Phosphorus-32, Radon-222, Radium 223, Radium-224, Rhenium-186, Rheniurn-188, Rubidium-82, Samarium-153, Selenium-75, Sodium-22, Sodium-24, Strontium-89, Technetium-99m, Thallium-201, Xenon-127, Xenon-133, Yttrium-90, Zirconium-89 and combinations thereof.

Where combinations of radioisotopes are used with the microparticles, preferred combinations of radioisotopes include the combination of one or more beta emitters with one or more gamma emitters. Examples of preferred combinations include, but are not limited, to Y-90/In-111Y-90/Tc-99m, Y-90/Ga-68, Y-90/F-18, Y-90/Cu-64, Cu-67/Cu-64, Y-90/Lu-177, P-32/In-111, P-32/Tc-99m, P-32/Ga-68, Ho-166/In-111, Ho-166/Tc-99m, Sm-153/In-111, and Sm-153/ Tc-99m.

Particularly preferred radioisotopes include Technetium-99m and Indium-111 (radio diagnostic gamma emitters), Lutetium-177 (being both a beta and gamma emitter), Samarium-153 and Yttrium-90 (radio therapeutic beta emitters) and Fluorine-18 and Galium-68 (diagnostic positron emitters). Tc-99m has been used for imaging and function studies of the brain, myocardium, thyroid, lungs, liver, gallbladder, kidneys, bone, blood, and tumors. Indium-111 pentetreotide has been used in imaging of neuroendocrine tumors that overexpress somatostatin receptors and has become standard for localization of these tumors. This radio ligand is internalized into the cell and can induce receptor-specific cytotoxicity by emission of Auger electrons. Lutetium-177 having both gamma and beta properties enables its use in imaging as well as treatment. It has a shorter radius of penetration than Y-90 which makes it an ideal candidate for radiotherapy of small tumors. Samarium-153 Lexidronam (chemical name Samarium-153-ethylene diamine tetramethylene phosphonate, abbreviated Sarnariurn-153 EDTMP, trade name Quadramet) is a complex of a radioisotope of the lanthanide element samarium with the chelator EDTMP. It has been used to treat cancer pain when cancer has spread to the bone. Once injected into a vein, it distributes throughout the body and localizes in areas where cancer has invaded the bone, allowing the beta particles (electrons) to destroy the nearby cancer cells. It is also commonly used in lung cancer, prostate cancer, breast cancer, and osteosarcoma. Yttrium-90 has been used in the treatment of various cancers including lymphoma, leukaemia, ovarian, colorectal, pancreatic, and bone cancers, and in treatment of rheumatoid arthritis by radionuclide synovectomy.

Although an attempt is made to provide an exhaustive list, it is well-known to nuclear medicine specialists that radioisotopes may be produced using a generator system like Mo-Tc or Sn/In systems, a thermal neutron reactor, a cyclotron, or fission produced. Accordingly, any radioisotopes with functional equivalents to those listed are intended to be encompassed wherever appropriate within the scope of the present invention.

In one preferred embodiment, the radioisotope is selected from the group consisting of Technetium-99m, Indium-111, Lutetium-177, Samarium-153, Yttrium-90, gallium-68 and fluorine 18.

Because the microparticle binds radio isotopes in a very simple reaction, the step of bonding the radioisotope to the strontium phosphate microparticle may be carried out just prior to the time of administration to a patient (e.g. within 6, 12 or 24hrs).

The invention also provides a strontium phosphate radio microparticle, prepared according to the process described herein.

The strontium phosphate microparticles achieve a porosity, or surface area, that allows for a significant amount of radioisotope to be bound. It is contemplated that surface area values of 90 square meters per gram or greater are within the scope of the present invention. The surface area may be up to to 200 square meters per gram, or greater.

Thus the present invention also provides microparticles, particularly microspheres, comprising crystalline strontium phosphate and having a diameter of between 5 and 1000 µm (preferably 5, 10 or 20 to 200µm). Preferably such microspheres have a surface area of 90 meters square per gram or greater.

Significantly, prior art attempts using calcium apatite to create the microparticles have provided much lower surface area values of only 40sq. meters per gram, or less. Further, these calcium-only microparticles demand complex manufacturing that includes a two-step process to adsorb the isotope requiring a binder, a heating step that destroys the surface area, and chemical precipitation.

These radioactive substantially spherical strontium phosphate radiomicroparticles are made by reacting a pre-made strontium-containing borate glass microparticle with a phosphate solution in amounts and for a sufficient time under suitable conditions to convert, partially or fully, the strontium-containing borate glass microparticle to a crystalline strontium phosphate microparticle. Once the glass has been converted and the porous material is made, a radioisotope-bearing radiopharmaceutical is then adsorbed or bonded to the substantially pure strontium phosphate microparticle and is then suitable for radio imaging and/or radiotherapy in a mammal.

The phosphate solution conversion process converts a solid strontium borate glass microparticle into a porous strontium phosphate material that is crystalline. The glass can be converted completely thus forming a completely porous or even hollow microparticle. The glass can also be partially converted thus resulting in a glass core and an outer porous strontium phosphate layer surrounding the glass core, to which radio isotopes may be bound or adsorbed The conversion of the borate glass is performed by exposing it to an aqueous phosphate solution. Many different phosphate solutions are contemplated as within the scope of the present invention. One non-limiting example includes phosphate buffered saline (PBS). PBS may be prepared in many different ways. Some formulations do not contain potassium, while others contain calcium or magnesium. Generally, PBS contains the following constituents: 137 mM NaCl, 2.7 mM KCl, 10 mM sodium phosphate dibasic, 2 mM potassium phosphate monobasic and a pH of 7.4. Another non-limiting example is a 0.25 M K₂PO₄ solution. Non-saline phosphate solutions may be prepared using monosodium phosphate (NaH₂PO₄), disodium phosphate (Na₂HP0₄), and water, with phosphoric acid or sodium hydroxide to adjust the pH as desired. Other concentrations and types of aqueous phosphate solutions are contemplated as within the scope of the invention.

Conversion of the strontium borate glass results, at the molecular level, in a high surface-area porous material, that itself, is an agglomeration containing the strontium phosphate compound. The pores of the surface provide access to the strontium phosphate compound. When a radioisotope is mixed with the microparticles, a strong chemical bond is made with the exposed strontium phosphate compound. Without being held to any particular chemical reaction or theory, it is believed that the isotope can bind in a substitution reaction removing a phosphate (PO₄) group, or it may be bound into a void space or it may substitute for a strontium ion (Sr²⁺).

Porosity may be determined by a number of methods well known in the art, however the preferred method is nitrogen absorption. These particles are preferably not biodegradable. A biodegradable particle will, not be present in the body after 2,4,or preferably 6 months.

By manufacturing a non-radioactive solid strontium-containing borate glass microparticle of a specific diameter, the size and shape of the strontium phosphate microparticle can be controlled; conversion results in a porous strontium phosphate microparticle of a specific diameter. Since the starting material is a solid strontium-containing borate glass microparticle and it becomes fully (or partially) converted to a porous strontium phosphate microparticle, the physical parameters of shape, size, diameter are dictated by the glass microparticle manufacturing process. Importantly, the size and dimension of the converted strontium microparticle are substantially the same as the size and dimension of the starting strontium borate glass microparticle. This feature provides the significant advantage of being able to control the size and dimension of the delivery vehicle itself, the porous strontium phosphate microparticle.

"Microparticle", as used herein, generally refers to a particle of a relatively small size, but not necessarily in the micron size range; the term is used in reference to particles of sizes that can be less than 50 nm to 1000 microns or greater. "Radio microparticle" refers to the microparticles of the present invention with one or more radioisotopes adsorbed thereon. The microparticles are preferably round spheroids having a preferred diameter of about 20 µm and above. In other preferred embodiments, the microparticles range from about 20 µm to about 200 µm, from about 30-80 µm, from about 20-40 µm, and from about 25 µm to 38 µm. In another embodiment, the diameter of the particles is from about 5 to about 100 microns, preferably from about 10 to about 50 microns. As used herein, the microparticle encompasses microspheres, microcapsules, ellipsoids, fibres, and microparticles, unless specified otherwise.

The porosity of the resulting strontium phosphate material and the controllable size and number of the microparticles provide an excellent delivery platform for delivering radiation to specific locations.

Importantly, no radioisotope is incorporated in the borate glass microparticle, thus, the process of manufacturing of the microsphere has been divorced from the process of adding the radioisotope label or the radio therapeutic. Prior radio microspheres must be manufactured as glass or biopolymer particles with the radioisotope as a homogeneous integral component of the glass or biopolymer. The present inventive approach provides a medical radiology professional the ability to control the radio diagnostic and radio therapeutic regimen by allowing them, in the clinical setting, to decide the type and quantity of radiopharmaceutical(s) to incorporate into the delivery vehicle.

The combination of the significantly increased surface area and the electrical attraction of the radioisotope to the porous microparticle provides for bonding multiple radioisotopes to the microparticle. In preferred embodiments, two radioisotopes are bound. Binding a first isotope to the porous microparticle is performed using simple mixing in an appropriate solution over a pre-determined time, and then washing and eluting out the unbound isotope. This provides a composition where a radioisotope is bound to the microparticles.

Binding a second isotope to the porous microparticle is performed by simple mixing in a solution having the second isotope. The second isotope does not displace the first isotope since the microparticles have a large surface area, and a nuclear pharmacist or other professional can take advantage of the different binding capacities of various radioisotopes to the microparticles. Thus, three and even four different radioisotopes can be bound within a single dose, or batch, of microparticles.

The invention is also useful in a method of administering strontium-phosphate radiomicroparticles to a patient in need thereof, comprising delivering, for example by catheter or injection, to a tissue target or organ of the patient, a composition comprising strontium-phosphate radio microparticles and a physiologically acceptable carrier.

In one preferred example, the method is a method of treatment of a tumor, particularly a hyper vascular tumour. Such treatments, as described further below, may be by delivery into a blood vessel such as to lodge the particles in the vasculature (radio embolization) or by direct injection into the site. Radiomicroparticles of the invention for use in such treatments are also provided by the invention.

In other preferred methods, there are provided additional features available singularly and in combination.

In one preferred method, the tissue target or organ is selected from the group consisting of: brain, myocardium, thyroid, lung, liver, spleen, gall bladder, kidney, bone, blood, and bead and neck, prostate, breast, ovarian and uterine.

Also disclosed herein is a method of obtaining a radiologic image of a tissue or organ of a patient, comprising administering to a tissue target or organ of the patient a composition containing strontium-phosphate radiomicroparticles, as described herein, and obtaining a radiologic image of the tissue or organ, typically by capturing the gamma radiation emitted by the radiomicroparticles. Specifcally, the invention provides a method for obtaining a radiologic image of a tissue or organ of a patient comprising obtaining a radiologic image of the tissue or organ of the patient to which a pharmaceutical composition comprising an aforementioned radio microparticle of the invention and a pharmaceutically acceptable carrier or diluent has been administered.

Diagnostic agents comprising the radiomicroparticles of the invention are thus also provided by the invention.

In this embodiment, the radioisotope is typically a radio diagnostic agent and in one embodiment is preferably Technetium-99m for SPECT imaging and fluorine 18 or Gallium 68 for PET imaging.. Typically for these methods, the radio microspheres will comprise spheres in the between about 20 and about 40 microns in diameter

The radiologic image may be captured using any suitable nuclear imaging technique.

Further, by using radiation dosimeters which show the keV peaks of various radioisotopes, activity can be tested, and tailored to a specific therapy. For example, treatment could in one non-limiting example consist of 100 units of isotope #1 and 50 units of isotope #2.

The microparticles may be administered to the patient in a variety of ways such as via catheters or needles and may be delivered alone or in combination with vaso constricting agents or by any other means of administration that effectively causes the microparticles to become embedded in the cancerous or tumor bearing tissue. For purposes of administration, the microparticles are preferably suspended in a pharmacologically acceptable suspension medium. The medium advantageously has a sufficient density or viscosity in order to prevent the microparticles from settling out of suspension during the administration procedure. Presently preferred liquid vehicles for suspension of the microparticles include polyvinylpyrrolidone (PVP such as that sold under the trade designation Plasdone K-30 and Povidone by GAF Corp), contrast media (such as that sold under the trade designation Metrizamide by Nyegard & Co. of Oslo, Norway or under the trade designation Renografin 76 by E. R. Squibb & Co.) and saline or mixtures thereof. Although many contrast media provide for the adjustment of specific gravity, the addition of specific gravity adjusting components, such as dextrans (e.g. 50% dextran) may also be useful.

The strontium containing borate glass microspheres described herein represent a further aspect of the present invention. These microspheres are preferably non biodegradable. The microspheres may be prepared from a homogenous mixture of powders (i.e., the batch) that is melted to form the desired glass composition. The exact chemical compounds or raw materials used for the batch is not critical so long as they provide the necessary oxides in the correct proportion for the melt composition being prepared. For instance, for making a strontium borate glass, then strontium, borate, and/or soda, powders may be used as some the batch raw materials.

Typically the strontium containing borate glass will comprise 10 or more mol% strontium oxide, but preferably 15mol% or greater. The strontium containing borate glass may comprise up to 25mol% strontium oxide, but more preferably 20±5mol% strontium oxide.

The present invention therefore provides a strontium containing borate glass microsphere, comprising 10 or more mol% strontium oxide and having a diameter of between 5 and 1000microns.

Typically the strontium containing borate glass will comprise 10mol% or more sodium oxide, preferably 15mole% or more. Preferably the strontium containing borate glass will comprise up to 30mol% sodium oxide. Preferably the the strontium containing borate glass will comprise 20±5 or 20±10mol% sodium oxide. Up to 25% of the sodium oxide portion of the glass may be replaced by lithium oxide or potassium oxide (or a combination of the two).

Typically the strontium containing borate glass will comprise at least 50% boron oxide, preferably the strontium containing borate glass will comprise at least 60% boron oxide, preferably the strontium containing borate glass will comprise up to 70% boron oxide. Preferably the strontium containing borate glass will comprise 60±10mol% boron oxide.

Typical compositions suitable for preparing the batch are given below, although it is to be understood that these should not be considered to be limiting; starting glasses having a wide range of compositions can be used so long as they contain a source of strontium oxide (from the glass) and phosphate, which may be from the starting glass or may be present in the solution in which the glass is being reacted. The general compositions given below assumes that the starting glass composition is being reacted in a solution that is the source of phosphate to form the strontium phosphate particle.

A typical composition comprises:
20±10mol% Na₂O which may contain up to 25% of Li₂O or K₂O or a combination thereof, on a molar basis,
20±5mol% SrO, up to 25% of which may be substituted by an alternative radiopaque oxide, such as barium, calcium manganese or cobalt oxides.
60±10mol% B₂O₃; and
0-5mol% of other oxides.

An example composition is 20:20:60 mol% Na₂O:SrO:B₂O₃

Thus the composition can be modified by varying the soda content by up to plus/minus 5 or 10mol%, and or the boron oxide content by plus/minus 10 mol%. It is also possible to substitute up to 5mol% Li₂O or K₂O (or a combination of the two) for a portion of the soda. It is also possible to substitute small amounts of P₂O₅ (say up to 5mol%) for a portion of the B₂O₃. A few (up to 5) mol% of a wide range of other oxides can be substituted into the base glass composition for various specific purposes such as varying the melting temperature or the conversion reaction rate, modifying the radio-opacity, etc. If needed, the SrO could be replaced partially (up to 5mol% of the 20) by barium oxide, calcium oxide, manganese oxide or cobalt oxide (eg CO) or a combination thereof.

The purity of each raw material is preferably greater than 99.9%. After either dry or wet mixing of the powders to achieve a homogeneous mixture, the mixture may be placed in a platinum crucible for melting. High purity alumina crucibles can also be used if at least small amounts of alumina can be tolerated in the glass being made. The crucibles containing the powdered batch are then placed in an electric furnace which is heated *1000.degree. to 1600.degree.* C., depending upon the composition. In this temperature range, the batch melts to form a liquid which is stirred several times to improve its chemical homogeneity. The melt should remain at 1000 degrees to 1600 degrees Celcius until all solid material in the batch is totally dissolved, usually 4-10 hours being sufficient. Significantly, by not incorporating the radioisotope into the melt, no radioisotope can be vaporized, thus avoiding a radiation hazard.

Another advantage of the invention is the ability to use radioisotopes that have a shorter half-life. For example, Tc-99m (Technetium-99m) cannot be made part of the glass, i.e. the half-life may often be too short to be useful when it is incorporated as part of certain homogeneous glass-radioisotope compositions. Additionally, the ability to use radioisotopes that would otherwise be destroyed or degrade by the glass-melt process. For example, trying to incorporate Technetium or Rhodium into a melt would vaporize the Technetium or Rhodium.

When melting and stirring is complete, the crucible is removed from the furnace and the melt is quickly quenched to a glass by pouring the melt onto a cold steel plate or into clean, distilled water. This procedure breaks the glass into fragments, which aids and simplifies crushing the glass to a fine powder. The powder is then sized and spheroidized for use.

To obtain spheroid microparticles having a diameter in the desired range of micrometres, the glass is processed using varying techniques such as grinding and passing through mesh sieves of the desired size, where the glass particles may be formed into spheroids by passing the sized particles through a gas/oxygen flame where they are melted and a spherical liquid droplet is formed by surface tension. A vibratory feeder located above the gas/oxygen burner slowly vibrates the powder into a vertical tube that guides the falling powder into the flame at a typical rate of 5 - 25 gm/hr. The flame is directed into a metal container which catches the spheroidized particles as they are expelled from the flame. The droplets are rapidly cooled before they touch any solid object so that, their spherical shape is retained in the solid product.

After spheroidization, the glass spheres are preferably collected and rescreened based upon size. As a non-limiting example, when the microparticles are intended to be used in the treatment of liver cancer, the fraction less than 30 and greater than 20 micrometres in diameter is recovered since this is a desirable size for use in the human liver. After screening, the -30/+20 microparticles are examined with an optical microscope and are then washed with a weak acid (HCl, for example), filtered, and washed several times with reagent grade acetone. The washed spheres are then heated in a furnace in air to 500 - 600 degree C. for 2-6 hours to destroy any organic material.

The final step is to examine a representative sample spheres in a scanning electron microscope to evaluate the size range and shape of the spheres. The quantity of undersize spheres is determined along with the concentration of non-spherical particles. The composition of the spheres can be checked by energy dispersive x-ray analysis to confirm that the composition is correct and that there is an absence of chemical contamination. The glass microparticles are then ready for phosphate conversion, bonding with radionuclide, and subsequent administration to the patient.

In accordance with the present invention, the above processing steps are merely exemplary and do not in any way limit the present invention. Similarly, the present invention is not limited to glass microparticles having a size described above; the size of the microparticles of the present invention may be varied according to the application.

The microparticles of the present invention may be used in a variety of clinical situations, and treatments for which internal radiation therapy is indicated. These include, for example, the treatment of tumors and the treatment of arthritis.

The treatment of both benign and cancerous tumors is contemplated. Tumors may be treated, for example, by selective internal radiation therapy (SIRT) for *i.a.,* hypervascular tumors or other tumors of areas that have favorable vasculature, including the liver (liver tumors include, for example, hepatocellular carcinomas or metastatic disease arising from other tissues, such as colorectal cancers), spleen, brain, kidney, head & neck, uterine, ovarian and prostate. The microparticles may be delivered via the vasculature, such as by catheter, to provide a radioembolization of the tissue, or they may be directly injected to provide a local depot. The microparticles may also be used for imaging, including a Liver/ Spleen scan - for tumors, cysts or hepatocellular disease; a brain scan- for tumors, trauma, or dementia; a Tumor Scan for malignant tumors or metastatic disease of the Kidney, Head & Neck, Uterine/Gynaecological; and any Scan or Therapy having favourable vasculature for this approach.

Since most organs, besides the liver, have only one blood vessel that feeds them, administration may be performed by delivery to that main feeder artery and allowing the microparticles to lodge in the capillary bed since they are too large to move through the capillary. The liver may require a specialized delivery regimen. In another embodiment, the vessel that feeds the tumor may be identified, and this artery is used to deliver the microparticles in a more local fashion.

### Figures

Figure 1 illustrates the conversion of a strontium containing borate glass microsphere into a crystalline strontium phosphate microsphere

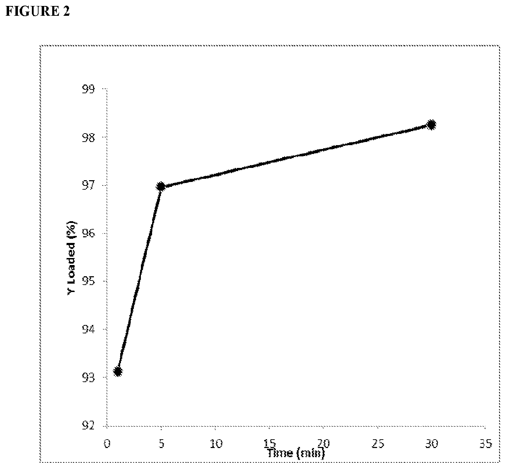

Figure 2 shows the up-take of ⁹⁰Y from a solution of ⁹⁰Yttrium chloride into strontium phosphate microspheres of the invention over a period of 30 minutes.

### Examples

### Example 1: Preparation of Strontium Phosphate Microspheres

Strontium containing borate glass microspheres were prepared from a batch comprising 20 mol%Na₂O, 20 mol%SrO and 60 mol% Ba₂O₃ as described above. The microspheres ranged in size from 44 to 105 microns.

The microspheres were reacted with 0.25molar K₂HPO₄ solution (pH=12) for lh, 6h, and 72 hrs at 85°C. After rinsing and drying, the surface area was measured by nitrogen absorption using a Micromeretics Tristar 3000 device. Multiple samples were measured and averaged. The measured surface area was 10, 93, and 72 m²/gm, respectively.

### Example 2: Loading Strontium Phosphate Microspheres with radioisotopes

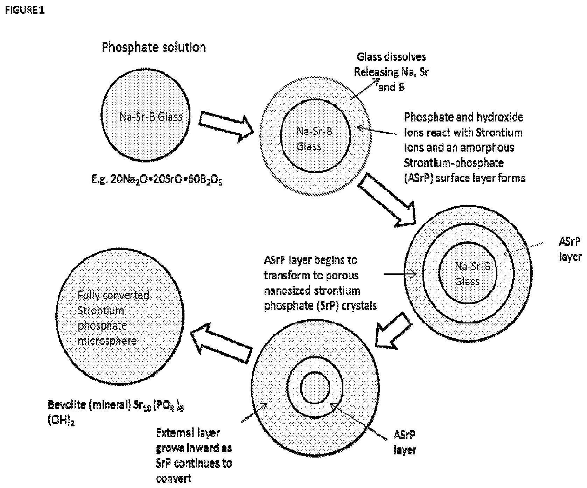

A solution of 5ug yttrium (Y-90) chloride in 0.5ml of 0.05N HCl was added to 15mg of microspheres and incubated for 30 minutes. The amount of yttrium loaded into the microspheres was determined at 1, 5 and 30 minutes in 5 replicates and averaged. Up-take of the yttrium with time is shown in fig. 1.

As various changes could be made in the above methods and products, without departing from the scope of the invention, it is intended that all matter contained in the above description or shown in any accompanying drawings shall be interpreted as illustrative and not in a limiting sense.

## Claims

1. A radio microparticle comprising:
crystalline strontium phosphate at least the surface layer of which consists of non-radioactive crystalline strontium phosphate and at least one radioisotope suitable for radio imaging and/or radiotherapy bonded or adsorbed to the surface thereof.

2. A radio microparticle of claim 1, wherein the surface layer is at least 0.5 µm thick.

3. A radio microparticle according to claim 1, wherein the whole microparticle is non-radioactive crystalline strontium phosphate.

4. The radio microparticle of any preceding claim, having a surface area of greater than about 90 square meters per gram.

5. The radio microparticle of any preceding claim which is a microsphere.

6. The radio microparticle of any preceding claim having a diameter of 5 µm to 1000 µm.

7. The radio microparticle of claim 1, wherein the radioisotope bonded or adsorbed to the surface is a therapeutic alpha or beta-emitting radioisotope.

8. The radio microparticle of claim 1, wherein the radioisotope bonded or adsorbed to the surface is a diagnostic positron or gamma-emitting radioisotope.

9. The radio microparticle of claim 1, wherein the radioisotope bonded or adsorbed to the surface comprises a combination of a therapeutic alpha or beta-emitting radioisotope and a diagnostic gamma or positron-emitting radioisotope.

10. The radio microparticle of claim 1, wherein the radioisotope bonded or adsorbed to the surface is selected from the group consisting of Technetium-99m, Indium-111, Lutetium-177, Samarium-153, Yttrium-90, Holmium-166, Gallium-68, Fluorine-18 and combinations thereof.

11. The radio microparticle of claim 10, wherein the radioisotope bonded or adsorbed to the surface is Technetium-99m.

12. The radio microparticle of claim 1, having at least two different radioisotopes bonded or adsorbed to the surface

13. A pharmaceutical composition comprising a radio microparticle of any of claims 1 to 12 and a pharmaceutically acceptable carrier or diluent.

14. A process for the preparation of a radio microparticle, comprising:
(i) reacting a strontium-containing borate glass microparticle with a phosphate solution such as to convert at least a portion of the strontium-containing borate glass to crystalline strontium phosphate; and
(ii) bonding or adsorbing at least one radioisotope suitable for radio imaging and/or radiotherapy to said strontium phosphate microparticle.

15. A process according to claim 14 wherein the radioisotope is bonded or adsorbed to the surface of the microparticle by contacting the microparticle with a solution of the radioisotope.

16. A process according to claim 14, wherein the radioisotope is selected from the group consisting of Technetium-99m, Gallium 68, Holmium -166, Fluorine-18, Indium-111, Yttrium-90, Lutetium-177, Samarium-153, and combinations thereof.

17. A method for obtaining a radiologic image of a tissue or organ of a patient comprising obtaining a radiologic image of the tissue or organ of the patient to which a pharmaceutical composition according to claim 13 has been administered.

18. The method of claim 17, wherein the tissue target or organ is selected from the group consisting of brain, myocardium, thyroid, lung, liver, spleen, gall bladder, kidney, bone, blood, head and neck, prostate, breast, and uterine.

19. The method of claim 17, wherein the radiologic image is obtained by single photon emission computed tomography (SPECT).

20. The method of claim 17, wherein the radiologic image is obtained by PET.

21. A radio microparticle according to claims 1 to 12 or a pharmaceutical composition according to claim 13, for use in a method of radiation therapy.

22. A radio microparticle for use according to claim 21 for use in the treatment of atherosclerosis or the treatment of a tumor.

## Patentansprüche

1. Radiomikropartikel, umfassend:
kristallines Strontiumphosphat, von dem mindestens die Oberflächenschicht aus nichtradioaktivem kristallinem Strontiumphosphat und mindestens einem an die Oberfläche davon gebundenen oder adsorbierten, für die Radiobildgebung und/oder Radiotherapie geeigneten Radioisotop besteht.

2. Radiomikropartikel nach Anspruch 1, wobei die Oberflächenschicht eine Dicke von mindestens 0,5 µm aufweist.

3. Radiomikropartikel nach Anspruch 1, wobei das ganze Mikropartikel nichtradioaktives kristallines Strontiumphosphat ist.

4. Radiomikropartikel nach einem der vorhergehenden Ansprüche mit einer Oberfläche von mehr als etwa 90 Quadratmetern pro Gramm.

5. Radiomikropartikel nach einem der vorhergehenden Ansprüche, bei dem es sich um ein Mikrokügelchen handelt.

6. Radiomikropartikel nach einem der vorhergehenden Ansprüche mit einem Durchmesser von 5 µm bis 1000 µm.

7. Radiomikropartikel nach Anspruch 1, wobei es sich bei dem an die Oberfläche gebundenen oder adsorbierten Radioisotop um ein therapeutisches alpha- oder beta-emittierendes Radioisotop handelt.

8. Radiomikropartikel nach Anspruch 1, wobei es sich bei dem an die Oberfläche gebundenen oder adsorbierten Radioisotop um ein diagnostisches Positronen- oder gamma-emittierendes Radioisotop handelt.

9. Radiomikropartikel nach Anspruch 1, wobei das an die Oberfläche gebundene oder adsorbierte Radioisotop eine Kombination eines therapeutischen alpha- oder beta-emittierenden Radioisotops und eines diagnostischen gamma- oder Positronen-emittierenden Radioisotops umfasst.

10. Radiomikropartikel nach Anspruch 1, wobei das an die Oberfläche gebundene oder adsorbierte Radioisotop aus der aus Technetium-99m, Indium-111, Lutetium-177, Samarium-153, Yttrium-90, Holmium-166, Gallium-68, Fluor-18 und Kombinationen davon bestehenden Gruppe ausgewählt ist.

11. Radiomikropartikel nach Anspruch 10, wobei es sich bei dem an die Oberfläche gebundenen oder adsorbierten Radioisotop um Technetium-99m handelt.

12. Radiomikropartikel nach Anspruch 1 mit mindestens zwei verschiedenen an die Oberfläche gebundenen oder adsorbierten Radioisotopen.

13. Pharmazeutische Zusammensetzung, umfassend ein Radiomikropartikel nach einem der Ansprüche 1 bis 12 und einen pharmazeutisch unbedenklichen Träger oder ein pharmazeutisch unbedenkliches Verdünnungsmittel.

14. Verfahren zur Herstellung eines Radiomikropartikels, umfassend:
(i) die Umsetzung eines strontiumhaltigen Boratglasmikropartikels mit einer Phosphatlösung zur Umwandlung mindestens eines Teils des strontiumhaltigen Boratglases in kristallines Strontiumphosphat und
(ii) die Bindung oder Adsorption mindestens eines für die Radiobildgebung und/oder Radiotherapie geeigneten Radioisotops an das Strontiumphosphatmikropartikel.

15. Verfahren nach Anspruch 14, wobei das Radioisotop durch Inkontaktbringen des Mikropartikels mit einer Lösung des Radioisotops an die Oberfläche gebunden oder adsorbiert wird.

16. Verfahren nach Anspruch 14, wobei das Radioisotop aus der aus Technetium-99m, Gallium-68, Holmium-166, Fluor-18, Indium-111, Yttrium-90, Lutetium-177, Samarium-153 und Kombinationen davon bestehenden Gruppe ausgewählt ist.

17. Verfahren zum Erhalt einer radiologischen Abbildung eines Gewebes oder Organs eines Patienten, bei dem man eine radiologische Abbildung des Gewebes oder Organs des Patienten, dem eine pharmazeutische Zusammensetzung nach Anspruch 13 verabreicht wurde, aufnimmt.

18. Verfahren nach Anspruch 17, wobei das Zielgewebe oder -Organ aus der aus Hirn, Myokard, Schilddrüse, Lunge, Leber, Milz, Gallenblase, Niere, Knochen, Blut, Kopf und Hals, Prostata, Brust und Uterus bestehenden Gruppe ausgewählt ist.

19. Verfahren nach Anspruch 17, wobei die radiologische Abbildung durch Einzelphotonen-Emissionscomputertomografie (Single Photon Emission Computed Tomography, SPECT) erhalten wird.

20. Verfahren nach Anspruch 17, wobei die radiologische Abbildung durch PET erhalten wird.

21. Radiomikropartikel nach einem der Ansprüche 1 bis 12 oder pharmazeutische Zusammensetzung nach Anspruch 13 zur Verwendung in einem Strahlentherapieverfahren.

22. Radiomikropartikel zur Verwendung nach Anspruch 21 zur Verwendung bei der Behandlung von Atherosklerose oder der Behandlung eines Tumors.

## Revendications

1. Radiomicroparticule comprenant :
du phosphate de strontium cristallin, au moins la couche de surface duquel étant constituée de phosphate de strontium cristallin non radioactif et au moins un radioisotope approprié pour une radioimagerie et/ou une radiothérapie lié ou adsorbé à la surface de celui-ci.

2. Radiomicroparticule selon la revendication 1, la couche de surface étant d'une épaisseur d'au moins 0,5 µm.

3. Radiomicroparticule selon la revendication 1, la microparticule entière étant du phosphate de strontium cristallin non radioactif.

4. Radiomicroparticule selon une quelconque revendication précédente, possédant une aire de surface supérieure à environ 90 mètres carrés par gramme.

5. Radiomicroparticule selon une quelconque revendication précédente qui est une microsphère.

6. Radiomicroparticule selon une quelconque revendication précédente possédant un diamètre de 5 µm à 1 000 µm.

7. Radiomicroparticule selon la revendication 1, le radioisotope lié ou adsorbé à la surface étant un radioisotope thérapeutique émetteur alpha ou bêta.

8. Radiomicroparticule selon la revendication 1, le radioisotope lié ou adsorbé à la surface étant un radioisotope diagnostique émetteur de positrons ou émetteur gamma.

9. Radiomicroparticule selon la revendication 1, le radioisotope lié ou adsorbé à la surface comprenant une combinaison d'un radioisotope thérapeutique émetteur alpha ou bêta et d'un radioisotope diagnostique émetteur de positrons ou émetteur gamma.

10. Radiomicroparticule selon la revendication 1, le radioisotope lié ou adsorbé à la surface étant choisi dans le groupe constitué par le technétium-99m, l'indium-111, le lutétium-177, le samarium-153, l'yttrium-90, l'holmium-166, le gallium-68, le fluor-18 et des combinaisons correspondantes.

11. Radiomicroparticule selon la revendication 10, le radioisotope lié ou adsorbé à la surface étant le technétium-99m.

12. Radiomicroparticule selon la revendication 1, possédant au moins deux différents radioisotopes liés ou adsorbés à la surface.

13. Composition pharmaceutique comprenant une radiomicroparticule selon l'une quelconque des revendications 1 à 12 et un support ou un diluant pharmaceutiquement acceptable.

14. Procédé pour la préparation d'une radiomicroparticule, comprenant :
(i) la mise en réaction d'une microparticule de verre de borate contenant du strontium avec une solution de phosphate de sorte à convertir au moins une partie du verre de borate contenant du strontium en phosphate de strontium cristallin ; et
(ii) la liaison ou l'adsorption d'au moins un radioisotope approprié pour une radioimagerie et/ou une radiothérapie à ladite microparticule de phosphate de strontium.

15. Procédé selon la revendication 14, le radioisotope étant lié ou adsorbé à la surface de la microparticule par mise en contact de la microparticule avec une solution du radioisotope.

16. Procédé selon la revendication 14, le radioisotope étant choisi dans le groupe constitué par le technétium-99m, le gallium-68, l'holmium-166, le fluor-18, l'indium-111, l'yttrium-90, le lutétium-177, le samarium-153, et des combinaisons correspondantes.

17. Procédé pour l'obtention d'une image radiologique d'un tissu ou d'un organe d'un patient comprenant l'obtention d'une image radiologique du tissu ou de l'organe du patient auquel une composition pharmaceutique selon la revendication 13 a été administrée.

18. Procédé selon la revendication 17, le tissu ou l'organe cible étant choisi dans le groupe constitué par le cerveau, le myocarde, la thyroïde, le poumon, le foie, la rate, la vésicule biliaire, le rein, un os, le sang, la tête et le cou, la prostate, le sein, et l'utérus.

19. Procédé selon la revendication 17, l'image radiologique étant obtenue par tomographie par ordinateur par émission monophotonique (SPECT).

20. Procédé selon la revendication 17, l'image radiologique étant obtenue par TEP.

21. Radiomicroparticule selon les revendications 1 à 12 ou composition pharmaceutique selon la revendication 13, pour une utilisation dans un procédé de thérapie par un rayonnement.

22. Radiomicroparticule pour une utilisation selon la revendication 21 pour une utilisation dans le traitement de l'athérosclérose ou le traitement d'une tumeur.
